# EUROPÄISCHE PATENTSCHRIFT

(11) **EP 1 742 682 B1**
(45) Veröffentlichungstag und Bekanntmachung des Hinweises auf die Patenterteilung: **23.12.2015**
(21) Anmeldenummer: 05736542.1
(22) Anmeldetag: 26.04.2005
(51) Int. Cl.: A61M 1/00, A61B 17/00, A61B 18/00

(54) **ANORDNUNG ZUM ENTFERNEN VON ABPRODUKTEN BEI DER ABLATION VON BIOLOGISCHEM GEWEBE**
ARRANGEMENT FOR THE REMOVAL OF WASTE PRODUCTS DURING THE ABLATION OF BIOLOGICAL TISSUE
DISPOSITIF POUR ELIMINER DES DECHETS LORS DE L'ABLATION DE TISSUS BIOLOGIQUES

(30) Priorität: 30.04.2004 DE 102004021680
(43) Veröffentlichungstag der Anmeldung: 17.01.2007
(73) Patentinhaber: Carl Zeiss Meditec AG, 07745 Jena (DE)
(72) Erfinder: DRESSLER, Georg, 07546 Gera (DE); MÖBIUS, Axel, 07745 Jena (DE)
(74) Vertreter: Geyer, Fehners & Partner
(86) Internationale Anmeldenummer: PCT/EP2005/004434
(87) Internationale Veröffentlichungsnummer: WO 2005/105173

(56) Entgegenhaltungen:
- WO-A-93/16741
- WO-A-99/66843
- DE-A1- 4 404 252
- US-A- 5 015 243
- US-A- 5 156 618
- US-A- 5 709 675
- US-B1- 6 267 752
- US-B1- 6 440 109
- US-B1- 6 494 965
- US-B1- 6 663 610
- US-B1- 6 755 817

## Beschreibung

Die Erfindung bezieht sich auf eine Anordnung zu Entfernen von Abprodukten, wie Rauch und Gewebepartikel, welche bei der Ablation von biologischem Gewebe mit Hilfe von Laserstrahlung entstehen.

Es ist bekannt, biologisches Gewebe durch Einbringen von Laserenergie ohne wesentliche thermische Schädigung der Zielgebiete abzutragen. Dieser quasi nichtthermische Prozess wird beispielsweise in der Medizin zur Bearbeitung von Knorpel, Zahnhartgewebe, Hautarealen und auch bei der Augenchirurgie zur Formung der Hornhaut genutzt (Photorefraktive Keratektomie PRK). Derartige Verfahren und zugehörige Vorrichtung sind beispielsweise in DE 197 27 573 C1 und EP 0 412 789 B1 veröffentlicht. Ein weiteres Verfahren, bei dem die Hornhaut mittels Laser bearbeitet wird, ist die Laser in Situ Keratomileusis (LASIK). Im Unterschied zur PRK wird hierbei zunächst ein Einschnitt in die Hornhaut vorgenommen und ein sogenannter Flap erzeugt, welcher während der Laserbearbeitung von dem Bearbeitungsgebiet weggeklappt wird. Nach Abschluss der Ablation, die damit quasi im Homhautinneren erfolgt, wird der Flap wieder zurückgeklappt.

Nachteilig ist, dass die bei der Abtragung des Gewebes entstehenden Abprodukte in Form von Rauch oder Gewebepartikeln die Luftqualität in unmittelbarer Nähe des Behandlungsortes beeinträchtigen, was einerseits zur Geruchsbelästigung für den Patienten und das behandelnde Personal führt, andererseits aber auch dazu, dass die Laserstrahlung partiell geschwächt wird. Letzteres ist insbesondere bei der photorefraktiven Keratektomie von Bedeutung, bei der die Hornhautoberfläche durch präzisen Materialabtrag geformt wird. Hier kommt es neben einer unbehinderten, feinfühligen Ausrichtbarkeit des Laserstrahles auch darauf an, dass die Strahlungsenergie bei exakt gleichbleibender Intensität in die Hornhaut eingebracht wird, damit das Ablationsergebnis in der gewünschten Qualität erzielt werden kann. Die Intensität aber wird durch abziehende und dabei den Laserstrahl durchquerende Rauchwolken und Gewebepartikel beeinflusst, was zu irregulären und somit unerwünschten Veränderungen der Ablationen führen kann. Bei der LASIK besteht zusätzlich die Gefahr, dass sich abgesprengte Gewebepartikel auf dem Flap niederschlagen und damit zu dessen Verunreinigung und zur Verschlechterung der optischen Qualität führen.

In der US-Patentschrift 5,344,418 ist eine Anordnung beschrieben, bei der ein Applikator nahe der Austrittsöffnung für den Laserstrahl Strömungskanäle für Gase bzw. für Luft aufweist, aus denen während der Behandlung ein Gas- bzw. Luftstrom auf das behandelte Gewebe gerichtet sind, was wie beabsichtigt zur Folge hat, dass die störenden Ablations-Abprodukte vom Behandlungsareal weggeblasen werden.

Hiermit wird das Problem der Luftverunreinigung und der Geruchsbelästigung für Patient und Operateur nicht gelöst.

Die genannten Nachteile vermag auch die mit der US-Patentschrift 5,181,916 dargelegte technische Lösung nicht zu beseitigen. Hier ist zwar kein Gasstrom in der Weise auf das behandelte Areal gerichtet, dass die Verunreinigungen weggeblasen werden, sondern die am Behandlungsort entstehenden Rauchgase werden mit Hilfe des Gasstromes abgesaugt. Hierzu dient eine Ansaugöffnung, die konzentrisch um die Mündungsöffnung, durch die der Laserstrahl austritt, angeordnet ist. Diese Lösung hat den Nachteil, dass das behandelte Areal während der Behandlung nur eingeschränkt einsehbar ist, da dieses durch die Ansaugöffnungen teilweise verdeckt wird.

Aus der DE 100 20 522 A1 der Anmelderin ist bekannt, die AblationsAbprodukte über einen über dem Ablationsgebiet angeordneten Kanal abzusaugen, wobei gleichzeitig durch einen konzentrisch um den Absaugkanal angeordneten Zufuhrkanal Spülgas zugeführt wird. Auch hier verdeckt die Vorrichtung das Behandlungsgebiet.

Diesen Nachteil kann auch die DE 101 29 650 A1 nicht beheben, bei der von einem kreisförmigen, um das Ablationsgebiet herum angeordneten Strömungskanal radialsymmetrisch auf das Ablationsgebiet gerichtete Ausströmöffnungen ein Gasstrom auf dieses gelenkt wird. Dadurch treffen die Strömungen aufeinander und werden derart überlagert, dass eine Richtungsumkehr und damit eine radial nach außen gerichtete Strömung erzeugt wird, welche die Ablationsprodukte und den entstehenden Rauch mit sich reißt. Auch hiermit wird das Problem der Geruchsbelästigung für Patient und Operateur nicht gelöst

Von diesem Stand der Technik ausgehend besteht die Aufgabe der Erfindung darin, die durch die Ablations-Abprodukte verursachten unerwünschten Schwankungen der Laserstrahlungsintensität zu verringern und eine Verunreinigung der Umgebung des Ablationsgebietes zu vermeiden..

Diese Aufgabe wird erfindungsgemäß durch die Merkmale des unabhängigen Patentanspruchs gelöst, vorteilhafte Weiterentwicklungen sind in den abhängigen Ansprüchen beschrieben.

Erfindungsgemäß ist bei einer Anordnung der eingangs genannten Art vorgesehen, dass die Ausströmöffnungen so angeordnet sind, dass die Gasströme symmetrisch auf das Ablationsgebiet auftreffen und sich zu einem resultierenden Gastrom überlagern, welcher von den Ausströmöffnungen weggerichtet ist.

Im Rahmen dieser Erfindung liegt es weiterhin, wenn in Richtung des resultierenden Gasstromes mindestens eine Ansaugöffnung vorgesehen ist, welche zur Abführung des abgesaugten Gases dient, das dann die Abprodukte mitführt.

Der bei der Behandlung entstehende und vom Behandlungsort kommende Rauch bzw. die sich von dort entfernenden Gewebepartikel werden von der Gas- bzw. Luftströmung erfasst, bewegen sich in Richtung der Ansaugöffnung und werden von dort abtransportiert.

Neue Seite 3a

Die US 6 663 610 offenbart eine Luftzuführung mit einer Ausströmöffnung, welche eine laminare, parallele Strömung abgibt..

Die US 6 494 965 beschreibt eine Anordnung nach dem Oberbegriff von Anspruch 1, bei der ein Haupt-Luftstrom die Abprodukte aus dem Operationsgebiet entfernt und sich ein sekundärer Luftstrom in Nähe einer Absaugvorrichtung mit dem Haupt-Luftstrom überlagert, um das Vorbeiströmen der Abprodukte an der Absaugvorrichtung zu verhindern.

Bevorzugt sind die Ausströmöffnungen diffusorartig aufgeweitet, so dass das Gas mit geringer werdender Strömungsgeschwindigkeit aus diesen Ausströmöffnungen austritt und so eine unerwünschte Verwirbelung des Gasstromes vermieden wird.

In einer besonders bevorzugten Ausgestaltung der Erfindung ist als Gas Luft vorgesehen und die Ausströmöffnungen sind mit einem Luftverdichter oder mit einem mit Luft gefüllten Druckbehälter verbunden. Vorteilhaft sollten an dem Luftverdichter bzw. an dem Druckbehälter Mittel zur Regulierung des Druckes der zugeführten Luft und damit auch der Strömungsgeschwindigkeit vorhanden sein. Derartige Mittel, wie beispielsweise Druckminderventile, sind im Stand der Technik hinreichend bekannt und müssen hier nicht näher beschrieben werden. Des weiteren ist es vorteilhaft, den Gasstrom gezielt anzufeuchten, um ein schnelles Austrocknen des Gewebes am Ablationsgebiet zu verhindern.

Durch die Überlagerung der Luftströmung in der Nähe des Ablationsgebietes wird das Rauchgas und die Gewebepartikel mit dem Luftstrom schnell aus dem Ort der Laserstrahlung bewegt und anschließend abgeleitet, so dass die auf das Behandlungsareal auftreffende Energie bezüglich ihrer Intensität nicht oder nur in wesentlich geringerem Ausmaß als beim Stand der Technik von nichttransparenten Teilchen beeinträchtigt wird.

Bevorzugt ist weiterhin vorgesehen, dass der Gesamtquerschnitt der Ausströmöffnungen und der Gesamtquerschnitt der Absaugöffnungen, der Überdruck an den Ausströmöffnungen und der Unterdruck an den Absaugöffnungen wie auch die Strömungsgeschwindigkeiten in den Ausströmöffnungen und die Strömungsgeschwindigkeiten in den Absaugöffnungen so aufeinander abgestimmt sind, dass die je Zeiteinheit abgesaugte Luftmenge um einen Faktor, der zwischen 1,1 und 1,3 liegt, größer ist als die durch die Ausströmöffnungen zugeführte Luftmenge.

Mit der erfindungsgemäßen Anordnung wird äußerst effektiv erreicht, dass die Geruchsbelästigung für den Patienten und den behandelnden Arzt aufgehoben ist und die Energiedichte der Laserstrahlung durch das Freihalten von Rauchgasen bzw. Gewebepartikeln über die Zeitdauer der Operation hinweg weitestgehend gleichmäßig bleibt.

In einer weiteren bevorzugten Ausgestaltung der Erfindung kann eine Einrichtung zum abwechselnden Unterbrechen der auf das Gewebe treffenden Laserstrahlung einerseits und der Luftströmung andererseits vorgesehen sein. Damit wird vorteilhaft erreicht, dass nach jeweils einer Behandlungsphase, in der die Laserstrahlung auf das Gewebe gerichtet war und eine Teilablation erfolgt ist, eine Absaugphase folgt, während der in der bereits beschriebenen Weise Gas bzw. Luft zugeführt bzw. abgesaugt wird und dabei die Ablationsprodukte abtransportiert werden. Nach Ende dieser "Reinigungsphase" wird der Gas- bzw. Luftstrom unterbrochen und es schließt sich erneut eine Behandlungsphase an. Auch hierbei wird erreicht, dass die Ablationsprodukte abgesaugt werden, insofern keine Geruchsbelästigung entsteht und auch die Laserstrahlung frei von nichttransparenten Teilchen gehalten wird, so dass auf das Gewebe eine Strahlung mit gleichbleibender Energiedichte treffen kann.

Eine besonders bevorzugte Ausführungsform der Erfindung ergibt sich, wenn in der Nähe der Ausströmöffnungen bzw. der Absaugöffnungen Infrarotlichtquellen zur Beleuchtung der Umgebung des Ablationsgebietes angeordnet sind. Mit diesen Lichtquellen lässt sich z.B. in einfacher Art und Weise eine Beleuchtung der Iris zum Zwecke der Verfolgung der Pupillenbewegung realisieren, wie sie z.B. in der US 6,334,683 beschrieben ist, auf deren gesamte Offenbarung hiermit hingewiesen wird. Dadurch ergeben besonders kompakte Einheiten, welche sowohl die Entfernung der Abprodukte realisieren als auch zusätzliche Lichtquellen für diese Eye-Tracker genannten Vorrichtungen überflüssig machen. Damit vereinfacht sich der Aufbau eines solchen Behandlungsgerätes und es sind weniger Bauteile störend im Blickfeld des Operateurs.

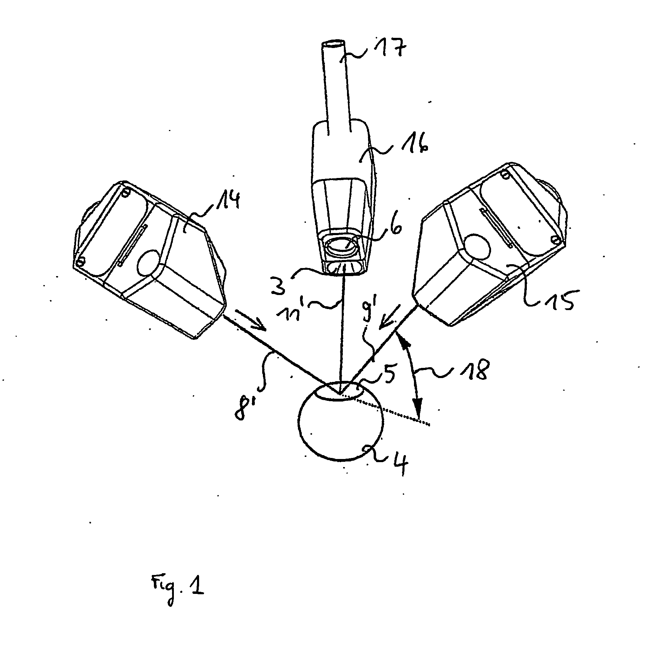

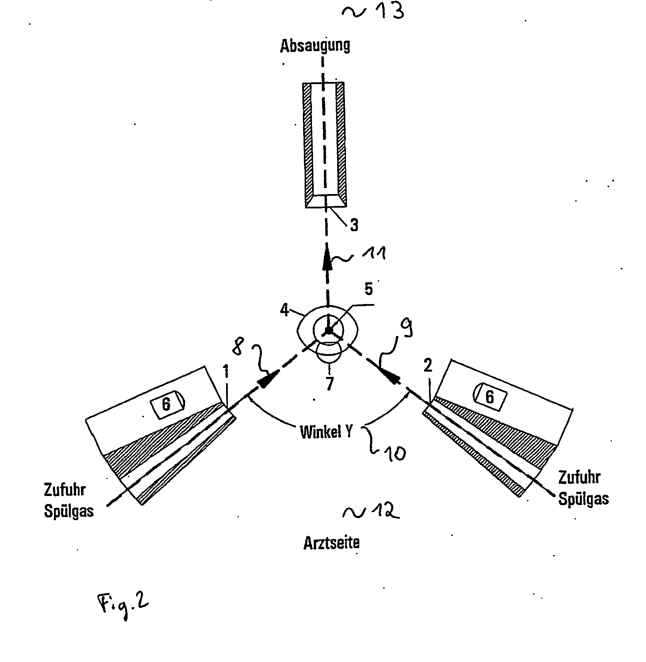

Die Erfindung soll nachfolgend anhand eines Ausführungsbeispieles näher erläutert werden. Die zugehörigen Zeichnungen zeigen in Fig. 1 eine Prinzipdarstellung der Erfindung als Draufsicht, in Fig. 2 als räumliche Ansicht.

In Fig. 1 sind zwei Ausströmöffnungen 1, 2 dargestellt, über die ein Spülgas auf das Ablationsgebiet geleitet wird, sowie eine Absaugöffnung 3, über die das Spülgas mit den darin gebundenen Ablationspartikeln abgesaugt wird. Als Ablationsgebiet 5 ist hier die Hornhaut eines Auges 4 dargestellt, von der ein Flap 7 geschnitten und zurückgeklappt wurde.

Die Strahlachsen 8, 9 der Spülgaszuführung schneiden die hier nicht dargestellte optische Achse des Behandlungslasers in der Arbeitsebene der Laserbehandlung. Diese optische Achse steht senkrecht auf der Zeichenebene von Fig. 1. Die beiden Gasströmungen 8, 9 überlagern sich beim Aufeinandertreffen und bilden eine vereinigte Strömung entlang der Achse 11, welche auf die Absaugöffnung 3 gerichtet ist.

Damit ergibt sich eine Y-förmige Anordnung der von der Arbeitebene beabstandeten Gasöffnungen zueinander, wobei das Y in Richtung 13 der Füße des Patienten gerichtet ist und die offene Seite 12 dem Operateur zugewandt ist.
In bevorzugten Ausgestaltung wird jeweils ein Gasstrom (0,5...10 m/s) über die Ausströmöffnungen 1 und 2 in Richtung des Auges 4 geleitet. Dabei weisen diese beiden Gasströme die gleichen Strömungsgeschwindigkeiten auf.
Die Ausströmöffnungen sind dabei so angeordnet, dass die Gasströmungen im Ablationsgebiet 5 aufeinander prallen und sich zu einer resultierenden Strömung in Richtung der Absaugöffnung 3 vereinigen. Die resultierende Strömung wird dann von der Absaugöffnung 3 aufgenommen.

Der durch das Aufeinandertreffen der Luftströmungen im Ablationsgebiet verursachte besonders intensive Gasdurchsatz führt zu einem Mitreißen der Partikel in der Strömung und damit zum effektiven Entfernen dieser aus dem Ablationsgebiet. Ablagerungen von Partikeln auf dem Flap 7 werden durch die Strömungsrichtung und den intensiven Gasdurchsatz vermieden. Auch eine durch die Partikel verursachte Geruchsbelästigung für Arzt und Patienten wird auf diese Weise minimiert.

In einer bevorzugten Ausführung wird die beschriebene Wirkung erreicht, wenn die Ausströmöffnungen einen Durchmesser von 6 mm aufweisen und die Gasströmung mit einer Geschwindigkeit von je 3 m/s auf das Ablationsgebiet geleitet wird. Der Saugunterdruck wird so abgestimmt, dass die resultierende Gasströmung an der Absaugdüse aufgenommen wird, z.B. mit einem Volumenstrom von 3 l/s.

Der Einfallswinkel der Gasströme gegenüber der Zeichenebene liegt im Bereich 40° ± 15°, und der Y-Winkel 10 beträgt 100° ± 20°. Die Größe der Winkel und die Anordnungen der Elemente garantieren, dass die Gasströmungen durch die Anatomie des Kopfes (Nase, Augenbrauen) möglichst wenig behindert werden.

Der Abstand zwischen Ausströmöffnungen 1, 2 bzw. Absaugöffnung 3 und dem Ablationsgebiet 5 beträgt in der bevorzugten Ausführung 75 mm ± 10 mm. Diese Abstände lassen dem behandelnden Arzt Bewegungsspielraum für nahezu ungehinderten Zugang zum Ablationsgebiet 5.

Baulich vereinigt mit den Ausströmöffnungen 1, 2 und der Absaugöffnung 3 sind Beleuchtungsquellen 6 für das Ablationsgebiet 5, welche bevorzugt als InfrarotLichtquellen ausgeführt werden und als Lichtquellen für eine hier nicht dargestellte Kamera, deren Bilder online zur Bestimmung des Pupillenortes und damit der Blickrichtung des Patienten ausgewertet werden können. Verfahren zu dieser Auswertung sind dem Fachmann bekannt und werden hier nicht näher erläutert, z.B. sei auf die US 6,334,683 hingewiesen.
Die dritte, in Fig. 1 nicht dargestellte Infrarotstrahlungsquelle 6 befindet sich oberhalb der Absaugöffnung 3.
Die IR-Strahlungsquellen sind dabei in einem Winkel von 120° zueinander angeordnet, wodurch die für das Eyetracking notwendige Infrarot-Strahlung ungehindert von anatomischen Gegebenheiten des Kopfes die Behandlungsebene erreichen und damit die Auswertbarkeit der von der Kamera aufgenommenen Bilder bedeutend erleichtert.
Durch das Anordnen der Ausströmöffnungen auf Arztseite wird einerseits der für die Strömung günstige Y-Winkel von 100° und andererseits der für optimale IR-Bestrahlung notwendige Winkel von 120° für die IR-Bestrahlungseinrichtungen ermöglicht.
Diese Anordnung der IR-Strahlungsquellen und Ausström- bzw. Absaugöffnungen zueinander sorgt durch einfache Zugänglichkeit zum Ablationsgebiet 5 für eine verbesserte Ergonomie für den Operateur und wird zusätzlich den funktionalen Erfordernissen der Beleuchtung für das Eyetracking sowie der Entfernung von Abprodukten aus dem Ablationsgebiet gerecht.

Die Ausström- und Absaugeinheiten sind durch entsprechend Führungen (hier nicht dargestellt) leicht zu befestigen und können daher zum Reinigen oder Sterilisieren ohne großen Aufwand entfernt werden.

In einer weiteren Ausgestaltung werden die Gasströme mit einer konstanten Temperatur und Luftfeuchtigkeit versehen. Das schließt auch die gezielte Anfeuchtung der Gasströmungen mit Ultraschallnebel ein, um noch konstantere Bedingungen im Ablationsgebiet zu erreichen.

Fig.2 zeigt die Anordnung aus Fig.1 in einer räumlichen Darstellung. Die beiden Einheiten 14 und 15 enthalten die hier nicht sichtbaren Ausströmöffnungen 1 und 2 und jeweils eine IR-Beleuchtungsquelle 6, welche auf das Ablationsgebiet 5 des Auges 4 gerichtet sind. Die Einheit 16 enthält die Absaugöffnung 3, welche mit einem Absaugkanal 17 verbunden ist und ebenfalls eine IR-Beleuchtungsquelle 6. Der Winkel 18 der Achsen 8', 9' und 11' der Beleuchtung gegenüber der (gedachten) Arbeitsebene 5 beträgt 40° ± 15°.

Die Ausströmöffnungen 1, 2 und die Absaugöffnung 3 stehen jeweils mit hier nicht dargestellten Anschlussstutzen in Verbindung, welche z.B. über Schläuche mit einem Verdichter oder Druckgasbehälter (die Öffnungen 1, 2 betreffend) bzw. einer Absaugvorrichtung (Öffnung 3 betreffend) verbunden sind.

Die Realisierung der Erfindung ist nicht an das dargestellte Ausführungsbeispiel gebunden, fachmännische Weiterentwicklungen führen nicht zu einem Verlassen des Schutzbereiches.

## Patentansprüche

1. Anordnung zum Entfernen von Abprodukten bei der Ablation von biologischem Gewebe der Hornhaut eines Auges eines Patienten durch Laserstrahlung mittels zugeführter Gasströmung in einem Ablationsgebiet (5), wobei zwei Ausströmöffnungen (1, 2) für die Gasströme und eine Absaugöffnung (3) vorgesehen sind, welche von einer Arbeitsebene, in der das Ablationsgebiet (5) liegt, beabstandet und Y-förmig so angeordnet sind, dass aus den Ausströmöffnungen (1, 2) ausströmende Gasströme symmetrisch im Ablationsgebiet (5) aufeinander treffen und sich dort zu einem resultierenden, von den Ausströmöffnungen (1, 2) weggerichteten resultierenden Gasstrom in Richtung der Absaugöffnung (3) überlagern, von welcher der Gasstrom aufgenommen wird, und **gekennzeichnet dadurch, dass** in der Nähe der Ausströmöffnungen (1, 2) und der Absaugöffnung (3) Beleuchtungsquellen (6) angeordnet sind, welche im Wesentlichen auf das Ablationsgebiet gerichtet und baulich mit den Ausströmöffungen (1, 2) bzw. der Absaugöffnung (3) vereinigt sind.

2. Anordnung zum Entfernen von Abprodukten nach Anspruch 1, wobei die Ausströmöffnungen (1, 2) diese unter einem gegenseitigen Winkel von 100° ± 20° auf das Ablationsgebiet gerichtet sind.

3. Anordnung zum Entfernen von Abprodukten nach Anspruch 2, wobei die Absaugöffnung (3) unter einem gegenseitigen Winkel von 130° ± 10° zu den Ausströmöffnungen (1, 2) angeordnet ist.

4. Anordnung zum Entfernen von Abprodukten nach einem der vorigen Ansprüche, wobei die Gasströme einem Winkel von 40° ± 15° mit einer durch das Ablationsgebiet bestimmten Ebene bilden.

5. Anordnung zum Entfernen von Abprodukten nach einem der vorigen Ansprüche, wobei der resultierende Gasstrom im Wesentlichen in Richtung des Körpers des Patienten gerichtet ist.

6. Anordnung zum Entfernen von Abprodukten nach einem der Ansprüche 1 bis 5, wobei die Beleuchtungsquellen (6) infrarotes Licht abstrahlen.

7. Anordnung zum Entfernen von Abprodukten nach einem der Ansprüche 1 bis 6, wobei die Beleuchtungsquellen (6) im Wesentlichen unter einem gegenseitigen Winkel von 120° angeordnet sind.

8. Anordnung zum Entfernen von Abprodukten nach einem der Ansprüche 1 bis 7, wobei die Beleuchtungsquellen (6) als Beleuchtung für einen Eyetracker dienen.

9. Anordnung zum Entfernen von Abprodukten nach einem der Ansprüche 1 bis 8, wobei der Abstand zwischen den Ausströmöffnungen (1, 2) bzw. der Absaugöffnung (3) und dem Ablationsgebiet (5) 75 ± 10 mm beträgt.

10. Anordnung nach einem der Ansprüche 1 bis 9, **dadurch gekennzeichnet, dass** der Gesamtquerschnitt der Ausströmöffnungen (1, 2) und derjenige der Absaugöffnung (3), der Überdruck an den Ausströmöffnungen (1, 2) und der Unterdruck an der Absaugöffnung (3) sowie die Strömungsgeschwindigkeiten in den Ausströmöffnungen (1, 2) und in der Absaugöffnung (3) so aufeinander abgestimmt sind, dass die je Zeiteinheit abgesaugte Luftmenge um das 1,1- bis 1,3-fache größer als die durch die Ausströmöffnungen (1, 2) zugeführte Luftmenge ist.

## Claims

1. An arrangement for the removal of waste products during the ablation of biological tissue of the cornea of a patient's eye by laser radiation, the said removal being performed by means of a gas stream fed to an ablation area (5), the arrangement featuring two discharge openings (1, 2) for the gas streams and a suction opening (3), which are located at some distance from an operating plane in which the ablation area (5) lies and arranged in a V configuration in such a way that gas streams emitted by the discharge openings (1, 2) meet symmetrically in the ablation area (5) and are superimposed there to form a resulting gas stream that is directed away from the discharge openings (1,2) and toward the suction opening (3), by which the gas stream is taken up; **characterized in that** light sources (6) are arranged in the vicinity of the discharge openings (1, 2) and the suction opening (3), these light sources being directed essentially at the ablation area and forming constructional units with the discharge openings (1, 2) or the suction opening (3), respectively.

2. An arrangement for removing waste products as claimed in Claim 1, with the discharge openings (1, 2) being directed at the ablation area at a mutual angle of 100° ± 20°.

3. An arrangement for removing waste products as claimed in Claim 2, with the suction opening (3) being arranged at an angle of 130° ± 10° relative to the discharge openings (1,2).

4. An arrangement for removing waste products as claimed in any one of the previous claims, with the gas streams forming an angle of 40° ± 15° with a plane defined by the ablation area.

5. An arrangement for removing waste products as claimed in any one of the previous claims, with the resulting gas stream being directed essentially toward the patient's body.

6. An arrangement for removing waste products as claimed in any one of Claims 1 through 5, with the light sources (6) radiating infrared light.

7. An arrangement for removing waste products as claimed in any one of Claims 1 through 6, with the light sources (6) arranged essentially at a mutual angle of 120°.

8. An arrangement for removing waste products as claimed in any one of Claims 1 through 7, with the light sources (6) serving to illuminate an eyetracker.

9. An arrangement for removing waste products as claimed in any one of Claims 1 through 8, with the discharge openings (1, 2) and the suction opening (3) having a distance from the ablation area (5) of 75 ± 10 mm.

10. An arrangement as claimed in any one of Claims 1 through 9, **characterized in that** the total cross-sectional area of the discharge openings (1, 2) and that of the suction opening (3), the excess pressure at the discharge openings (1, 2) and the vacuum at the suction opening (3) as well as the flow velocities in the discharge openings (1, 2) and in the suction opening (3) are matched to each other in such a way that the air volume sucked off per unit of time is 1.1. to 1.3 times the air volume fed through the discharge openings (1, 2)

## Revendications

1. Dispositif pour l'élimination de déchets lors de l'ablation de tissus biologiques de la cornée d'un oeil d'un patient par rayon laser à l'aide de l'introduction d'un flux de gaz dans une zone d'ablation (5), deux ouvertures de sortie de flux (1, 2) pour les flux de gaz et une ouverture d'aspiration (3) étant prévues, qui sont disposées à une certaine distance d'un plan de travail dans lequel se trouve la zone d'ablation (5) et sont disposées en Y de façon à ce que les flux de gaz sortant par les ouvertures de sortie de flux (1, 2) se rencontrent de manière symétrique dans la zone d'ablation (5) et s'y superposent en un flux de gaz résultant qui s'éloigne des ouvertures de sortie de flux (1, 2) en direction de l'ouverture d'aspiration (3), à partir de laquelle le flux de gaz est aspiré et **caractérisé en ce que**, à proximité des ouvertures de sortie de flux (1, 2) et de l'ouverture d'aspiration (3), se trouvent des sources d'éclairage qui sont dirigées essentiellement vers la zone d'ablation et sont intégrées physiquement dans les ouvertures de sortie de flux (1, 2) ou l'ouverture d'aspiration (3).

2. Dispositif pour l'élimination de déchets selon la revendication 1, les ouvertures de sortie de flux (1, 2) étant dirigées vers la zone d'ablation sous un angle mutuel de 100° ± 20°.

3. Dispositif pour l'élimination de déchets selon la revendication 2, l'ouverture d'aspiration (3) est disposée avec un angle mutuel de 130° ± 10° par rapport aux ouvertures de sortie de flux (1, 2).

4. Dispositif pour l'élimination de déchets selon l'une des revendications précédentes, les flux de gaz formant un angle de 40° ± 15° avec un plan défini par la zone d'ablation.

5. Dispositif pour l'élimination de déchets selon l'une des revendications précédentes, le flux de gaz résultant étant dirigé essentiellement en direction du corps du patient.

6. Dispositif pour l'élimination de déchets selon l'une des revendications 1 à 5, les sources d'éclairage (6) émettant de la lumière infrarouge.

7. Dispositif pour l'élimination de déchets selon l'une des revendications 1 à 6, les sources d'éclairage (6) étant disposées essentiellement avec un angle mutuel de 120°.

8. Dispositif pour l'élimination de déchets selon l'une des revendications 1 à 7, les sources d'éclairage (6) servant d'éclairage pour un « eyetracker ».

9. Dispositif pour l'élimination de déchets selon l'une des revendications 1 à 8, la distance entre les ouvertures de sortie de flux (1, 2) ou l'ouverture d'aspiration (3) et la zone d'ablation (5) est de 75 ± 10 mm.

10. Dispositif pour l'élimination de déchets selon l'une des revendications 1 à 9, **caractérisé en ce que** la section transversale totale des ouvertures de sortie de flux (1, 2) et celle de l'ouverture d'aspiration (3), la surpression au niveau des ouvertures de sortie de flux (1, 2) et la dépression au niveau de l'ouverture d'aspiration (3) ainsi que les vitesses d'écoulement dans les ouvertures de sortie de flux (1, 2) et dans l'ouverture d'aspiration (3) sont harmonisées entre elles de façon à ce que la quantité d'air aspirée par unité de temps soit 1,1 à 1,3 fois supérieure à la quantité d'air introduite par les ouvertures de sortie de flux (1, 2).
